# EUROPEAN PATENT SPECIFICATION

(11) **EP 1 251 794 B1**
(45) Date of publication and mention of the grant of the patent: **28.02.2007**
(21) Application number: 01900394.6
(22) Date of filing: 08.01.2001
(51) Int. Cl.: A61F 2/00

(54) **AREAL IMPLANT WITH X-RAY-VISIBLE ELEMENTS**
FLÄCHIGES IMPLANTAT MIT RÖNTGENSICHTBAREN ELEMENTEN
IMPLANT DE SUPPORT LOCAL AVEC ELEMENTS VISIBLES AUX RAYONS X

(30) Priority: 31.01.2000 DE 10004832
(43) Date of publication of application: 30.10.2002
(73) Proprietor: Ethicon GmbH, 22851 Norderstedt (DE)
(72) Inventor: PRIEWE, Jörg, 24114 Kiel (DE); SCHULDT-HEMPE, Barbara, 24576 Bad Bramstedt (DE); WALTHER, Christoph, 24568 Kattendorf (DE)
(74) Representative: UEXKÜLL & STOLBERG
(86) International application number: PCT/EP2001/000122
(87) International publication number: WO 2001/056499

(56) References cited:
- EP-A- 0 783 873
- EP-A- 0 797 962
- WO-A-90/03036
- FR-A- 2 712 177
- US-A- 5 741 327

## Description

The invention relates to an areal implant with a flexible polymer-based basic structure.

Areal implants with a flexible polymer-based basic structure, which are prepared for example in the form of meshes or tapes, are widely used. They are used for example in a surgical operation, in order to support or to reinforce an organ or tissue or to promote the healing procedure. Such an implant often has to remain permanently or at least for a very long time in the body of a patient. In this case, the basic structure contains non-resorbable polymer or only very slowly-resorbable polymer.

In the course of time, the inserted implant can shift, shrink or fold. This can lead to problems for the patient. Diagnostically, by means of imaging procedures, this can be detected, if at all, only with great difficulty, as conventional areal implants are relatively fine, in order to ensure a sufficient flexibility, and have already become fused with tissue a short time after the operation, so that they can hardly be made out, if at all, using conventional and widespread diagnostic methods such as ultrasound or X-ray procedures, so that no diagnostically usable conclusions are possible.

Metal meshes can be made visible with X-ray procedures, but are not sufficiently flexible.

In EP 0 894 481 temporary and removable X-ray-visible markers are described, which are essentially applied on stents. A stent is generally taken to be a tubular metal mesh or a perforated metal tube which is much more inelastic than an areal implant with a flexible basic structure on polymer base. The markers shown in this document are not suitable for an areal implant which is permanently inserted in the body of a patient.

A temporary marking with a resorbable marker which, as a rule, consists of a resorbable polymer and an element such as zirconium or iodine, is described in EP 0 894 503. The use of a resorbable and physiologically compatible polymer which is combined with toxic elements such as elementary zirconium or iodine, is problematical.

In WO 94/01056 stent implants are shown which consist of a tubular metal mesh and loop-shaped metal wires knotted onto them which are made from an X-ray-dense metal, such as tantalum. These implants are likewise relatively bulky, as they are meant to support vessels, and are not comparable with areal implants with a flexible polymer-based basic structure.

R. Sahagian (Critical Insight: Marking Devices with Radioopaque Coatings, MD&DI May 1999) describes X-ray-visible coatings on stents and catheters which are produced with the help of a sputtering process. These coatings are intended for short-term implants such as catheters or for not very flexible metal meshes such as stents and are unsuitable for permanently implanted flat polymer implants. Furthermore, sputtering processes have the disadvantage that often only a fraction of the expensive precious metals used is deposited on the implant.

Swabs marketed by the firm Hartmann under the name "Telacomp", contain a polypropylene thread filled with barium sulphate. However, these swabs are not proposed for permanent implantation. The marking only reveals whether a swab has remained in the body after an operation, but gives no information about the orientation of the swab in the body, as the thread filled with barium sulphate runs only in the middle of the swab and thus a shifting or a folding at the edge cannot be recognised. Barium sulphate is to be considered as critical for use in a long-term implant, because of the toxicity of barium ions, if it is not sufficiently encapsulated.

FR-A-2 712 177 discloses an areal implant woven or knitted from multifilament polyester yarn in a square or rectangular mesh structure. The implant can contain a contribution of polypropylene yarns. To increase the rigidity of the implant, the polyester yarns are combined in warp and/or weft with metallic wires which also establish a radiological marker. The features of the preamble of claim 1 are known from FR-A-2 712 177.

EP-A-0 783 873 discloses a radiopaque marker attached to one or more ends of a stent, wherein the radiopaque marker may be a tube disposed around a length of wire on the stent.

The object of the invention is to create a possibility, which is economical and safe for the patient, of being able to monitor a flexible areal implant which is implanted in the body of a patient for a long time or permanently, after the operation at any chosen time, in a way which does not burden the patient.

This object is achieved by an areal implant having the features of claim 1. Advantageous designs of the invention are given in the dependent claims.

The areal implant according to the invention has a flexible polymer-based basic structure and has X-ray-visible elements. The implant is preferably set up for permanent implantation, the X-ray-visible elements being present in tissue-compatible form, i.e. if at all possible releasing no toxic substances even after a long time, and being permanently connected to the basic structure. The implant is flexible as a whole. The X-ray-visible elements enable the implant to be made visible as required at any time after the surgical operation to insert the implant. It is particularly advantageous to have the X-ray-visible elements arranged in an areal pattern. This is because, in this case, a shift of the implant or of sections of the implant (e.g. folding of corner) can be easily recognised on the X-ray image. Shrinking or stretching is also visible through the changed distances between the individual components of the pattern.

At least some of the X-ray-visible elements have a polymer tube or a cord which is at least partly filled with particles of a size of at most 2.5 mm made from an X-ray-visible substance. For preference, the X-ray-visible substance is additionally fixed in the polymer tube or the cord, for example by thermal shrinking and/or gluing.

With the help of such a polymer tube or such a cord, an X-ray-visible substance can be introduced into the basic structure as a quasi-linear element. The implant is preferably so designed that the flexibility, the elasticity, the rigidity and the tensile strength are not adversely affected.

The basic structure preferably has a non-resorbable polymer so that it is suitable for a permanent implant, but can also have a proportion of resorbable polymer. Examples of tissue-compatible non-resorbable or very slowly resorbable substances are polyalkenes (e.g. polypropylene or polyethylene), fluorinated polyolefins (e.g. polytetrafluoroethylene or polyvinylidene fluoride), polyamides, polyurethanes, polyisoprenes, polystyrenes, polysilicones, polycarbonates, polyaryletherketones (PEEK), polymethacrylates, polyacrylates, aromatic polyesters, polyimides as well as mixtures and/or copolymers of these substances.

Polyhydroxy acids (e.g. polylactides, polyglycolides, polyhydroxybutyrates, polyhydroxyvaleriates), polycaprolactones; polydioxanones, synthetic and natural oligo- and polyaminoacids, polyphosphazenes, polyanhydrides, polyorthoesters, polyphosphates, polyphosphonates, polyalcohols, polysaccharides, polyethers, resorbable glasses as well as mixtures and/or copolymers of such substances, for example, come into consideration as resorbable substances.

The flexible basic structure is preferably designed as mesh, tape, foil or perforated foil and in principle it can be of conventional type. It is preferably thinner than 1 mm. It is conceivable that the shape of the implant to be used in a given surgical operation is cut to size from a larger piece of material before the operation.

X-ray-visible elements, which are particularly clearly visible in an X-ray procedure, contain a chemical element or several chemical elements of a medium or high atomic number in sufficient density. Non-toxic and chemically stable chemical elements or chemical compounds with these properties are particularly suitable. If no sufficient long-term stability is guaranteed, such as for example in the case of barium sulphate, additional measures are recommended, as explained further below. Examples of X-ray-visible substances which can be used in the X-ray-visible elements are pure zirconium dioxide, stabilized zirconium dioxide, zirconium nitride, zirconium carbide, tantalum, tantalum pentoxide, barium sulphate, silver, silver iodide, gold, platinum, palladium, iridium, copper, ferric oxides, not very magnetic implant steels, non-magnetic implant steels, titanium, alkali iodides, iodated aromatics, iodated aliphatics, iodated oligomers, iodated polymers as well as mixtures and alloys of such substances. Non-magnetic materials offer advantages because, for example, they do not disturb imaging diagnostic procedures using magnetic resonance.

In a preferred version of the invention, part of the X-ray-visible elements is formed as pre-shaped bodies of respective length, width and height in the range 0.1 mm to 50 mm, the pre-shaped bodies being attached to the basic structure.

The pre-shaped bodies can be present in many different shapes, e.g. as beads, balls, small tubes, rods, small plates, rings, discs, bones or clips. A coating or outer wrapper (preferably made from polypropylene or beeswax) made from a non-resorbable material can be advantageous, in particular if the long-term stability of an uncoated pre-shaped body is doubtful. Such a pre-shaped body can be permanently connected to the flexible basic structure in many different ways, in particular by knotting, compression, welding and/or bonding (e.g. with or with the help of foils, small foil strips, foil tubes, small meshes or adhesives), by attachment direct to the basic structure or to a holding device (such as e.g. a thread, a mesh, etc) connected to the basic structure.

The pre-shaped bodies (scattering bodies) can be designed and connected to the basic structure in many different ways. Citable as further examples are perforated bodies (e.g. sleeves, rings, beads) which are strung on a thread or attached to a thread and consist of an X-ray-visible substance, for example zirconium dioxide, barium sulphate, non-magnetic implant steel, titanium, gold or other precious metals. In addition, the pre-shaped bodies can be coated with one of the polymers used for the basic structure. In a preferred version, at least one pre-shaped body contains a mixture of at least one X-ray-visible substance with a binding agent, preferably a non- or slowly resorbable polymer and/or a wax. Furthermore, the X-ray-visible substances can be encapsulated in a glass. Pre-shaped bodies attached to a thread can be directly incorporated into the flexible basic structure during the manufacture of the implant or fixed on the basic structure by suitable thread connections or thermal fixing.

The pre-shaped bodies considered have the advantage that, because of their size, they can be also made visible with other diagnostic procedures such as ultrasound methods and magnetic resonance procedures. Furthermore, the flexibility of the implant is not, or is only slightly, impaired by the presence of the pre-shaped bodies. A correspondingly thick X-ray-visible monofilament thread or wire, the X-ray visibility of which corresponds to that of the pre-shaped bodies present at the implant, would on the other hand lead to a marked stiffening of the flexible basic structure, which is not desired. ,

Pre-shaped bodies designed as clips are particularly suitable for subsequent attachment to a flexible basic structure. Metal clips are considered for example, but also clips which are cast from a composite material which contains X-ray-visible substances in the form of chemical elements, oxides, salts or organic compounds which are compounded in a non-resorbable polymer.

A pre-shaped body can also be connected to the basic structure in an advantageous manner by covering it with thin foil pieces on both sides and attaching it to the basic structure via two-sided welding with ultrasound.

A particularly preferred type of attachment of the X-ray-visible elements to the basic structure is one which can be used for pre-shaped bodies in particular, and in which a pre-shaped body or several pre-shaped bodies (e.g, balls made from zirconium dioxide) are packed into short tubes, e.g. a few centimetres long, at equal intervals. If the basic structure is a mesh made from polypropylene, these tubes are preferably made from polyethylene or polypropylene for medical use and have, in the lumen, an equal or somewhat greater diameter than the pre-shaped bodies. These short, filled tubes are thermally fixed on the mesh, optionally under pressure. Short strips thus form, in which the X-ray-visible substance is completely encapsulated and which are so strongly fixed that these strips or the enclosed particles of the X-ray-visible substance cannot be removed from the mesh without destroying it. The mechanical properties of the basic structure, such as for example the flexural strength, are not adversely affected.

In a further preferred version of the invention, part of the X-ray-visible elements is formed as polymer, into which an X-ray-visible substance made from particles of a size in the range of 10 nm to 500 µm (preferably 10 nm to 100 µm) is compounded.

The polymer with X-ray-visible substance can be present in the implant for example in the form of monofilaments, filaments reduced along sections, multifilaments, twines, braided threads, cords, foils, films, small film tapes, tapes or such forms which are provided with knots. In advantageous designs, the basic structure has polymer with X-ray-visible substance, which for example can be incorporated direct into the basic structure (e.g. knitted in), or the polymer with X-ray-visible substance is attached to the basic structure as additional component; mixed forms are also conceivable. Preferred examples of the X-ray-visible substance of the polymer are zirconium dioxide as well as barium sulphate; in the latter case the polymer should be coated in addition with a non-resorbable polymer or wax, in order to prevent the barium sulphate, which has a toxic effect, from being released in the body of a patient in the long term.

Thus, for example, zirconium dioxide particles with an average diameter of less than 1 µm can be compounded particularly advantageously in polypropylene with a mass content of 10% to 90%, preferably 50%, and mono- and multifilaments can be extruded from this material. These can be incorporated into the basic structure in the form of threads, cords or thin tapes, so that cutting is possible for the surgeon, without the X-ray-visible elements coming loose from the supporting basic structure.

In the case of monofilament threads, care is to be taken that the threads, when they have good X-ray visibility, are also still sufficiently elastic in order to not adversely affect the flexural properties of the implant. This can be achieved by diluting a relatively thick X-ray-visible thread with a diameter of for example 0.2 mm to 2 mm at pre-set intervals by thermal treatment and stretching and optionally reacting it with plasticizers or plasticizing polymers (e.g. adding of polyethylene to polypropylene). Such X-ray-visible filaments are preferably incorporated into the basic structure so that they have no supporting function and cannot adversely affect the properties of the implant, which are essentially dictated by the basic structure, such as its tensile strength or elasticity.

Monofilaments or multifilaments made from polymer with X-ray-visible substance can be knotted once or repeatedly in the area of the basic structure. The local visibility in the X-ray image is thereby increased, without the flexural strength of the filaments being increased to an extent such as that which is present in a thread with the knot thickness. Furthermore, as a result of their size, such knots can also be detected with other diagnostic methods, such as ultrasound procedures or magnetic resonance.

It is particularly advantageous if part of the X-ray-visible elements has an X-ray-visible symbol which is preferably provided repeatedly and at equal intervals. Such a symbol can be sewn for example from X-ray-visible threads, stitched from X-ray-visible threads, embossed from X-ray-visible foil or put together from X-ray-visible objects or X-ray-visible powder.

The symbol is preferably neither point- nor mirror-symmetrical; thus the lower-case letter "e" can, for example, be considered. With the help of the symbol or the symbols, a deformation of the implant, e.g. an undesired knotting or overlapping, on the one hand from the relative distances between the symbols and on the other hand from a change in direction of the symbols can be recognised in the X-ray image. It is also conceivable to use the product name of the implant or the company name of the manufacturer as the symbol, e.g. in the form of embossed pieces made from X-ray-visible foil, which are attached at equal intervals to the basic structure.

It is of particular advantage if the implant in the body of the patient can also be detected with the help of ultrasound procedures and/or in magnetic resonance tomography. This can be achieved in particular with the help of larger pre-shaped bodies or knots, as already mentioned.

It is thus possible, using the invention, to provide implantable flexible polymer meshes and tapes with an X-ray-visible substance so that the desired properties, such as low weight, elasticity, tensile strength and behaviour at the implantation site, are not essentially altered and also other diagnostic procedures, such as X-ray photographs, computer tomography photographs of regions lying behind the implant or magnetic resonance procedures, are not noticeably disturbed by the marking with X-ray-visible elements. Furthermore, it is possible to anchor the marking so firmly to the basic structure so that no false diagnoses are effected by a migration of loosened X-ray-visible elements. Furthermore, the implant material can be marked such that it is possible for the doctor to cut it to size later without causing a loosening of the X-ray-visible elements, whereby size, position and shape of the implant would no longer be able to be detected with certainty. The marking with the X-ray-visible elements can be designed to be toxicologically harmless, and this to be effective for decades. Thus, not only proven inert substances such as e.g. gold or titanium (which develops a protective layer) are suitable, but also, in sufficiently and permanently encapsulated form e.g. barium sulphate, which is otherwise only permitted as an oral X-ray contrast medium because barium ions are very toxic and, despite the low solubility of barium sulphate of 2.5 µg/ml, toxic effects can be expected in the case of long-term implantation (Chang, Biomaterials 2, 151-155, 1981).

The invention as well as supplementary examples of X-ray-visible elements are illustrated in the following with the help of individual embodiments: The drawings show in
- Figure 1: a top view of the implant according to Example 1, which has clips made from titanium as X-ray-visible elements,
- Figure 2: a top view of the implant according to Example 9, which has balls made from zirconium dioxide as X-ray-visible elements,
- Figure 3: a schematic representation of the implant according to Example 14, which is produced on a crochet galloon machine and has X-ray-visible threads, and
- Figure 4: a thread course representation to illustrate the manufacture of the implant according to Example 15.

### Example 1

### Manufacture of an X-ray-visible, fine and coarse-pored polypropylene mesh with clips made from titanium

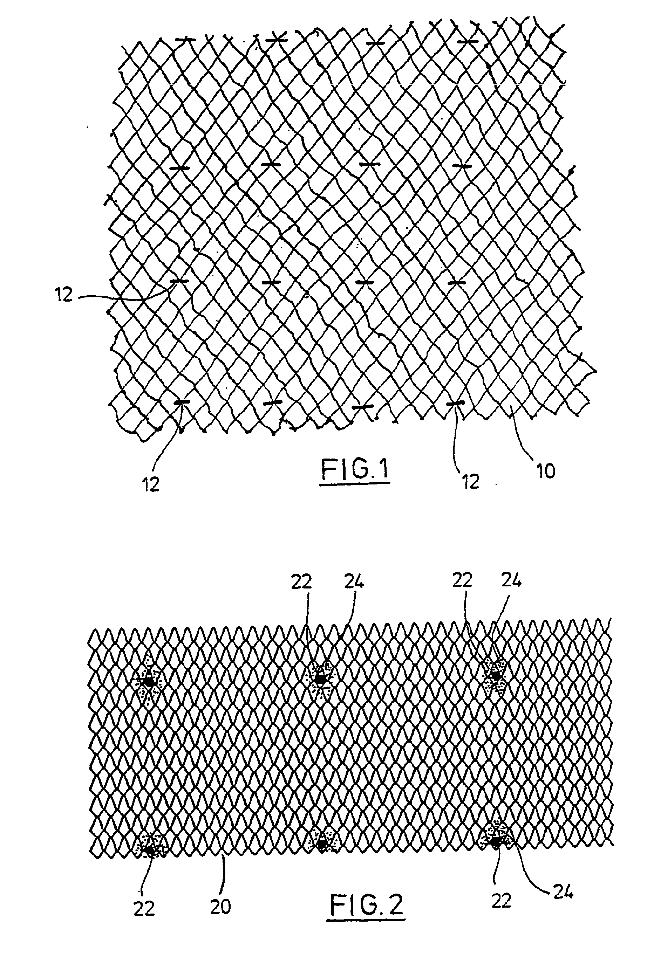

A partly-resorbable implant mesh customary in the trade, marketed by Ethicon GmbH under the name " Vypro", was boiled in a 10% soda solution, rinsed with water and air-dried in order to remove the resorbable part. Clips made from titanium ("LIGACLIP Extra", small, manufactured by Ethicon Endo-Surgery) were attached to the resulting fine, but coarse-pored polypropylene mesh on the intersection points of every seventh and eighth wale using the associated applicator. Figure 1 shows the basic structure 10 in the form of the polypropylene mesh as well as the clips designated by 12.

The implant made in this way was placed under a 10 cm-thick gel cushion for sonography, in order to have an absorption comparable to the in-vivo-situation, and X-rayed (focus-film distance: 1m, exposure 52/2.5). The X-ray-visible elements in the form of clips 12 were clearly visible in the X-ray image. They could not be separated from the mesh by gentle manual pulling.

### Example 2

### Manufacture of an X-ray-visible, fine and coarse-pored polypropylene mesh with clips made from implant steel

A partly-resorbable implant mesh customary in the trade ("Vypro", Ethicon GmbH) was boiled in a 10% soda solution, rinsed with water and air-dried, in order to remove the absorbable part. Implantable clips made from steel ("LIGACLIP Extra", small, stainless steel with the US-material number 316L, from Ethicon Endo-Surgery) were attached to the resulting fine, but coarse-pored polypropylene mesh (basic structure) on the intersection points of every seventh and eighth wale using the associated applicator.

The implant made in this way was placed under a 10 cm-thick gel cushion for sonography, in order to have a basic absorption comparable to the in-vivo-situation, and X-rayed (focus-film distance: 1m, exposure 52/2.5). The X-ray-visible elements in the form of the clips were easily visible in the X-ray image. They could not separated from the mesh by gentle manual pulling.

### Example 3

### Manufacture of an X-ray-visible, fine and coarse-pored polypropylene mesh with clips made from implant steel

The procedure was as in Example 2, but instead of the small clips, implantable clips of medium size made from stainless steel were used ("LIGACLIP Extra", medium-size, made from stainless steel with the US-material number 316L, from Ethicon Endo-Surgery). The clips could be very well recognised in the X-ray image.

### Example 4

### Manufacture of a mesh which is X-ray-visible, fine and coarse-pored, partly-resorbable and reinforced with medical steel tubes

Sleeves made from a medical chromium/nickel steel tube each with a length of 4 mm, an external diameter of 1.3 mm and an internal diameter of 1.0 mm were each fixed at 25 mm intervals, by compression at one end, on a monofilament (called " Pronova") manufactured by Ethicon Inc. which was 5.0 mil (0.127 mm) thick and made from fluorinated polyolefins. The fixed objects could not be moved on the monofilament manually. The monofilament prepared in this way was incorporated as a thread into an implant mesh customary in the trade ("Vypro", Ethicon GmbH) serving as a flexible basic structure and showed a very good contrast in the X-ray image.

### Example 5

### Manufacture of a polypropylene mesh which is X-ray-visible, fine and coarse-pored, reinforced with steel tubes made from non-magnetic implant steel

Steel sleeves made from non-magnetic implant steel ("Phynox", from Minitubes, Grenoble) each with a length of 4 mm, an external diameter of 1.2 mm and an internal diameter of 0.8 mm were fixed, by knotting in front of and behind each sleeve, on a thread made from polypropylene ("Prolene", 6.0 mil (0.1524 mm) diameter, Ethicon Inc.). The distance between each of the sleeves was approx 30 mm. These threads were drawn in between every fifth and sixth wale into a polypropylene mesh customary in the trade and showed a very good contrast in the X-ray image behind an approx. 5 cm-thick gel cushion.

### Example 6

### Marking of a polypropylene mesh with welded pre-shaped bodies made from zirconium dioxide as X-ray-visible elements

Balls made from stabilized zirconium dioxide (diameter 1.5 mm, YSZ-Ytt-stabilized, 95% ZrO₂, 5% Y₂O₃, Mühlmeier Mahltechnik) were placed on a polypropylene mesh customary in the trade ("Prolene-Netz", Ethicon GmbH) serving as basic structure, each at 3 cm intervals. Subsequently, circular pieces with a diameter of approx 1 cm were cut out of a 250 µm-thick polypropylene foil, and were pressed centered onto the balls and each welded with 8 to 10 weld points with a "USG 440" ultrasound welding probe manufactured by Lühr. Subsequently, foil pieces of the same size were welded onto the back of the mesh as counterpieces.

The pre-shaped bodies made from stabilized zirconium dioxide were very clearly visible in the X-ray image (very good contrast behind a 10 cm-thick gel cushion) and could not be separated from the implant mesh by manual pulling on the implant mesh, rubbing or repeated bending.

### Example 7

### Marking of a polypropylene mesh with welded pre-shaped bodies made from zirconium dioxide as X-ray-visible elements

The procedure was as in Example 6, but in contrast to this, balls with a diameter of 0.5 mm were used (YSZ-Ytt-stabilized, 95% ZrO₂, 5% Y₂O₃, Mühlmeier Mahltechnik), and a 50 µm-thick polypropylene foil was used as a foil. A still very good contrast was shown in the X-ray image, but slightly fainter than in Example 6.

### Example 8

### Manufacture of a polypropylene mesh with X-ray-visible symbols

Symbols in the shape of a lower-case letter "e" (each 5 mm long, 5 mm wide and approx. 0.5 mm thick) were sewn onto a mesh customary in the trade, made from polypropylene as a flexible basic structure ("Prolene", Ethicon GmbH), with a 0.127 mm-thick gold thread (Aldrich), each at 2.5 cm intervals. The X-ray-visible elements in the form of these letters could be well recognised in the x-ray image under a 10 cm-thick gel cushion.

### Example 9

### Marking of a polypropylene mesh with tube-reinforced balls made from zirconium dioxide

20 balls made from zirconium dioxide (0.5 mm diameter, YSZ-Ytt-stabilized, 95% ZrO₂, 5% Y₂O₃, Mühlmeier Mahltechnik) were attached by means of a polyethylene reinforcement to a polypropylene mesh customary in the trade (" Prolene" , Ethicon GmbH) as a basic structure which was cut to a size of 6 cm x 7 cm. To this end, each ball was packed centered into a 1.5 mm-long tube made from polyethylene with an external diameter of 1 mm and an internal diameter of 0.5 mm from Portex (England). The tube pieces were arranged on the mesh so that each interval between the individual balls was 1.5 cm. Subsequently, the mesh was kept in a heatable printing press with baking paper for 10 to 30 seconds at a temperature of 135°C and a pressure of 2 bar.

Afterwards, the balls were encapsulated in rounded film pieces and firmly anchored to the mesh so that they could not be removed by rubbing or tearing until the mesh was destroyed. Figure 2 illustrates a section of the prepared implant. The mesh (basic structure) is designated by 20 and the zirconium dioxide balls by 22. The resulting rounded film pieces are indicated by the dots 24 in the area surrounding the balls 22.

In the case of the mesh treated under the conditions described, no thermal shrinkage was noticed. The mesh had the same flexural strength and tensile strength as the original mesh not subjected to an increased temperature. The increase in weight caused by the zirconium dioxide balls serving as X-ray-visible elements was only 7%. The balls were very well visible in the X-ray image.

### Example 10

### Marking of an implant with encapsulated barium sulphate as an X-ray-visible substance

A 3.3 cm-long polypropylene tube (Portex) with an internal diameter of approx. 1.5 mm was filled with an approx 1 mm-thick barium sulphate-containing multifilament thread, (taken from the swab material "Telacomp" from Hartmann) with a length of 2.7 cm so that there was a tube overhang of approx 3 mm to the left and to the right. This tube was bent twice so that the shape of an angular letter "C" resulted, and was fixed with bees wax on a commercially available polypropylene mesh (" Prolene-Netz", 6 mil (0.1524 mm), Ethicon GmbH) and subsequently thermally welded, as described in Example 9.

An almost round membrane with a diameter of approx 2 cm developed, in the centre of which the " C" made from the multifilament filled with barium sulphate could be recognised. The coating remained stable even when forcefully and rapidly bent and rubbed with the fingernails. The X-ray-visible element in the form of the "C" showed a good X-ray contrast.

### Example 11

### Marking of a partly-resorbable mesh with zirconium dioxide and subsequent hydrolysis

10 balls made from stabilized zirconium dioxide (0.5 mm diameter, YSZ-Ytt-stabilized, 95% ZrO₂, 5% Y₂O₃, Mühlmeier Mahltechnik) were attached via a polyethylene reinforcement to a partly-resorbable polypropylene-containing mesh ("Vypro-Netz", 15 cm x 10 cm, Ethicon GmbH) as a flexible basic structure. To this end, each ball was centred in an approx. 2 to 3 cm-long polyethylene tube (Portex 800/110/160) with an external diameter of 1 mm and an internal diameter of approx. 0.5 mm. The tube pieces were arranged on the mesh so that each interval between the balls was 2.5 cm; each tube piece was placed diagonally over 6 wales and 24 stitch rows. Subsequently, the mesh was kept in a heatable printing press with baking paper for 30 seconds at a temperature of 137 °C and a pressure of 2.2 bar.

The balls were then encapsulated in rounded film pieces and firmly anchored to the mesh so that they could not be removed by gentle rubbing or tearing. Furthermore, the mesh showed no thermal shrinkage under these conditions. The mesh had almost the same flexural strength and tensile strength as the original mesh not subjected to a thermal treatment. The zirconium dioxide markings were very clearly visible in the X-ray image. They could not be removed from the mesh by gentle rubbing and repeated bending.

Subsequently, the resorbable part of the mesh was removed by being boiled for half an hour in a 10% soda solution. The resulting polypropylene mesh was rinsed with water and air-dried. The zirconium dioxide balls were still on the mesh and could not be removed from the mesh by gentle manual rubbing and repeated bending.

### Example 12

### Marking of a partly-resorbable mesh with zirconium dioxide in polypropylene reinforcement

The procedure was analogous to Example 11 but with the difference that a polypropylene tube (Portex "PP 60 x 100 FT", Lot G0515) served to reinforce the zirconium dioxide balls and the heatable printing press was kept for 3 minutes at a temperature of 121 °C and a pressure of 3 bar. The mesh showed practically no thermal shrinkage under these conditions. It had the same flexural strength and tensile strength as the original mesh. The markings in the form of zirconium dioxide balls could be very clearly seen in the X-ray image.

### Example 13

### Marking of a coarse-pored mesh with fine-pored marked mesh pieces

Circular pieces with a diameter of 1.4 cm were cut out round the zirconium dioxide balls from the mesh manufactured in Example 9. These round and small-pored mesh pieces were subsequently placed on a large-pored polypropylene mesh hydrolysed according to Example 1 (original material " Vypro" , see Example 1) and welded with a "USG 440" type Lühr ultrasound welding probe with 8 to 10 weld points in each case.

### Example 14

### Manufacture of a polymer mesh which contains X-ray-visible polymer threads

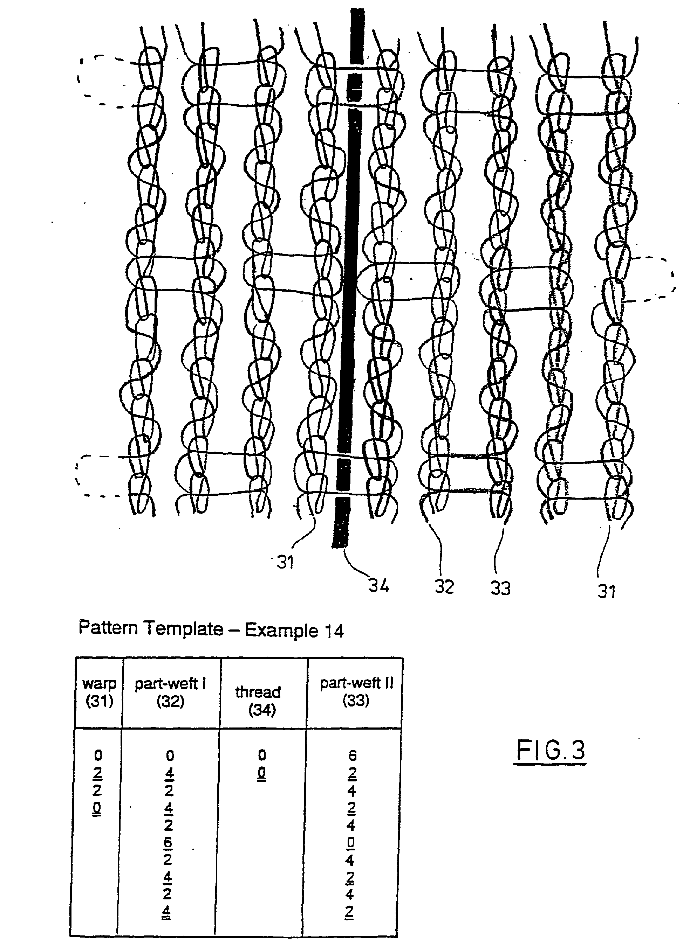

A polypropylene mesh was manufactured on a "RD3MT3/420 SN" type Müller crochet galloon machine as illustrated in Figure 3. Figure 3 also shows the corresponding pattern template. Like a conventional "Vypro" mesh manufactured by Ethicon GmbH, this mesh was prepared with two part-wefts 32, 33, however the open-pillar stitches technique was chosen for the warp numbered 31.

In addition, several X-ray-visible stationary threads 34 were incorporated into the mesh, which were prepared as multifilament polypropylene threads (300 tex) with a zirconium dioxide content of 50 wt.-%. The stationary threads 34 ran in the rapport of 10 wales between the two part-wefts 32 and 33 of the basic structure. As a meshing of these threads with the stationary threads 34 did not take place, the X-ray-visible stationary threads 34 were not responsible for the stretching and strength properties of the implant.

### Example 15

### Manufacture of an X-ray-visible knitted product

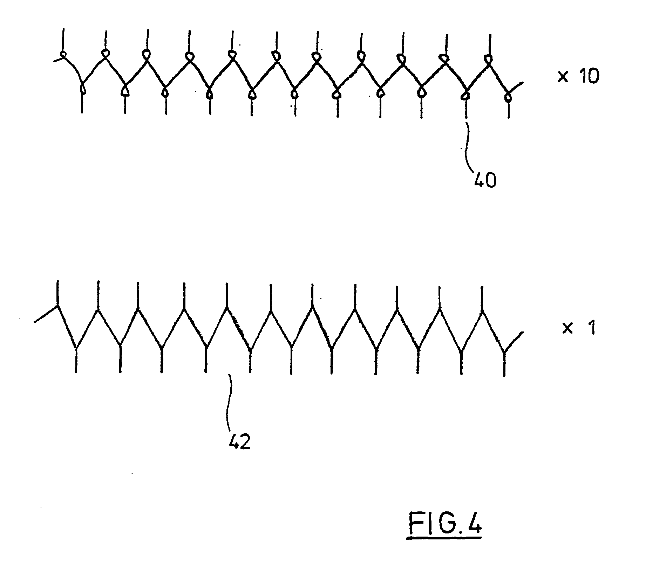

A right/right-knitted product was manufactured on a Shima Seiki 12-pitch flatbed-knitting machine basically from a 320-den polypropylene yarn. An X-ray-visible thread was incorporated into every 11^{th} stitch row. Each X-ray-visible thread, which was prepared from a polypropylene yarn (300 tex) containing 35 wt.-% zirconium dioxide pigment, therefore follows on 10 stitch rows 40 (see Figure 4) in an incorporated loop 42.

By using the different threads and the chosen pattern, the knitted fabric acquired a transverse texture, however with sufficient elasticity.

### Example 16

### Marking of a partly-resorbable mesh with zirconium dioxide balls in cords

A cord was braided on a customary ball-braiding machine with 36 bobbins from a polypropylene yarn (2 x 60 den polypropylene multifilament yarn per bobbin) with approx. 60 braids/inch. Zirconium dioxide balls each with a diameter of 1.5 mm were incorporated therein at a mutual interval of approx. 10 mm (zirconium dioxide balls as in Example 6). After thermofixing of the braided product, the cord was incorporated as stationary thread into a composite mesh consisting of "Vicryl"- and polypropylene fibres. ("Vicryl" is a trade name of Ethicon GmbH for a resorbable copolymer made from glycolide and lactide in a ratio of 9 to 1).

The implant obtained showed an only slightly higher flexural strength than an unaltered "Vypro" mesh. The zirconium dioxide balls of the implant were very clearly visible in the X-ray image.

### Example 17

### Marking of a non-resorbable mesh with cords made from threads filled with zirconium dioxide

A thread was manufactured on a customary ball braiding head with 8 bobbins from polypropylene multifilament yarn (60 den polypropylene multifilament yarn per bobbin) with approx. 60 braids/inch, into which zirconium dioxide balls with a diameter of 0.5 mm (zirconium dioxide balls as in Example 7) were incorporated, each at a 5 mm interval. After thermofixing the braided thread, the braided threads were incorporated into a knitwear product made from a non-resorbable, synthetic monofilament with a diameter of approx. 0.1 mm.

The implant showed no noticeable change in flexural strength compared with a knitted mesh without an incorporated X-ray-visible thread. The zirconium dioxide balls were clearly visible in the X-ray image.

### Example 18

### Marking of a non-resorbable mesh with knotted threads filled with zirconium dioxide

A thread, manufactured as in Example 17, however with only approx. 35 braids/inch, was knotted both in front of and behind each zirconium dioxide ball, in order on the one hand to prevent a displacement of the small balls, but on the other hand also to obtain a very soft braided thread. Subsequently, this very soft braided thread was incorporated into a knitted fabric as in Example 17 without previous thermofixing.

The flexural strength and elasticity of the implant was not measurably altered by the thread filled with zirconium dioxide balls. The zirconium dioxide balls placed in the implant were clearly visible in the X-ray image.

### Example 19

### Manufacture of a tape marked with X-ray-visible elements

A so-called striped braid was manufactured on a customary braiding head, threads filled with approx. 30 wt.-% zirconium dioxide being braided round as so-called cores. For the braid construction, the braid had 19 bobbins each with 3 x 60 den polypropylene multifilament yarn. The six cores each consisted of a polypropylene monofilament with a diameter of approx. 0.2 mm filled with approx. 30 wt.-% zirconium dioxide.

The tape obtained was approx. 6 mm wide, and the X-ray image showed the cores filled with the X-ray-visible zirconium dioxide as six fine bands.

## Claims

1. Areal implant with a flexible polymer-based basic structure and with X-ray-visible elements, wherein the implant is flexible as a whole, **characterized in that** at least part of the X-ray-visible elements comprises a polymer tube or a cord which is filled at least partly with particles of a size of at most 2.5 mm made from an X-ray-visible substances.

2. Implant according to claim 1, **characterized in that** the X-ray-visible elements are arranged in an areal pattern.

3. Implant according to claim 1 or 2, **characterized in that** the basic structure includes non-resorbable polymer.

4. Implant according to one of claims 1 to 3, **characterized in that** the basic structure has one of the forms chosen from the following group: meshes, tapes, foils, perforated foils.

5. Implant according to one of claims 1 to 4, **characterized in that** the X-ray-visible elements have at least one of the X-ray-visible substances chosen from the following group: pure zirconium dioxide, stabilized zirconium dioxide, zirconium nitride, zirconium carbide, tantalum, tantalum pentoxide, barium sulphate, silver, silver iodide, gold, platinum, palladium, iridium, copper, ferric oxides, not very magnetic implant steels, non-magnetic implant steels, titanium, alkali iodides, iodated aromatics, iodated aliphatics, iodated oligomers, iodated polymers, alloys of substances thereof capable of being alloyed.

6. Implant according to one of claims 1 to 5, **characterized in that** the X-ray-visible substance is additionally fixed in the polymer tube or the cord, preferably by thermal shrinking and/or gluing.

7. Implant according to one of claims 1 to 6, **characterized in that** the implant contains at least one of the substances chosen from the following group: polyalkenes, polypropylene, polyethylene, fluorinated polyolefins, polytetrafluoroethylene, polyvinylidenefluoride, polyamides, polyurethanes, polyisoprenes, polystyrenes, polysilicones, polycarbonates, polyaryletherketones, polymethacrylates, polyacrylates, aromatic polyesters, polyimides, copolymers of polymerisable substances thereof.

8. Implant according to one of claims 1 to 7, **characterized in that** the basic structure has a proportion of resorbable polymer, which preferably contains at least one of the substances chosen from the following group: polyhydroxy acids, polylactides, polyglycolides, polyhydroxybutyrates, polyhydroxyvaleriates, polycaprolactones, polydioxanones, synthetic and natural oligo- and polyaminoacids, polyphosphazenes, polyanhydrides, polyorthoesters, polyphosphates, polyphosphonates, polyalcohols, polysaccharides, polyethers, resorbable glasses, copolymers of polymerisable substances thereof.

9. Implant according to one of claims 1 to 8, **characterized in that** the implant is also detectable by means of ultrasound and/or magnetic resonance tomography.

## Patentansprüche

1. Flächiges Implantat, mit einer flexiblen Grundstruktur auf Polymerbasis und mit röntgensichtbaren Elementen, wobei das Implantat als Ganzes flexibel ist, **dadurch gekennzeichnet, dass** zumindest ein Teil der röntgensichtbaren Elemente einen Polymerschlauch oder eine Kordel aufweist, der bzw. die zumindest teilweise mit Teilchen einer Größe von höchstens 2,5 mm aus einer röntgensichtbaren Substanz gefüllt ist.

2. Implantat nach Anspruch 1, **dadurch gekennzeichnet, dass** die röntgensichtbaren Elemente in einem flächigen Muster angeordnet sind.

3. Implantat nach Anspruch 1 oder 2, **dadurch gekennzeichnet, dass** die Grundstruktur nicht-resorbierbares Polymer aufweist.

4. Implantat nach einem der Ansprüche 1 bis 3, **dadurch gekennzeichnet, dass** die Grundstruktur eine der aus der folgenden Gruppe ausgewählten Formen hat: Netze, Bänder, Folien, gelochte Folien.

5. Implantat nach einem der Ansprüche 1 bis 4, **dadurch gekennzeichnet, dass** die röntgensichtbaren Elemente mindestens eine der aus der folgenden Gruppe ausgewählten röntgensichtbaren Substanzen aufweisen: reines Zirkondioxid, stabilisiertes Zirkondioxid, Zirkonnitrid, Zirkoncarbid, Tantal, Tantalpentoxid, Bariumsulfat, Silber, Silberiodid, Gold, Platin, Palladium, Iridium, Kupfer, Eisenoxide, wenig magnetische Implantatstähle, unmagnetische Implantatstähle, Titan, Alkaliiodide, iodierte Aromaten, iodierte Aliphaten, iodierte Oligomere, iodierte Polymere, Legierungen von legierfähigen Substanzen davon.

6. Implantat nach einem der Ansprüche 1 bis 5, **dadurch gekennzeichnet, dass** die röntgensichtbare Substanz in dem Polymerschlauch oder der Kordel zusätzlich fixiert ist, vorzugsweise durch thermisches Schrumpfen und/oder Verkleben.

7. Implantat nach einem der Ansprüche 1 bis 6, **dadurch gekennzeichnet, dass** das Implantat mindestens eine der aus der folgenden Gruppe ausgewählten Substanzen aufweist: Polyalkene, Polypropylen, Polyethylen, fluorierte Polyolefine, Polytetrafluorethylen, Polyvinylidenfluorid, Polyamide, Polyurethane, Polyisoprene, Polystyrole, Polysilikone, Polycarbonate, Polyaryletherketone, Polymethacrylate, Polyacrylate, aromatische Polyester, Polyimide, Copolymere von polymerisierbaren Substanzen davon.

8. Implantat nach einem der Ansprüche 1 bis 7, **dadurch gekennzeichnet, dass** die Grundstruktur einen Anteil an resorbierbarem Polymer aufweist, das vorzugsweise mindestens eine der aus der folgenden Gruppe ausgewählten Substanzen aufweist: Polyhydroxysäuren, Polylactide, Polyglykolide, Polyhydroxybutyrate, Polyhydroxyvaleriate, Polycaprolactone, Polydioxanone, synthetische und natürliche Oligo- und Polyaminosäuren, Polyphosphazene, Polyanhydride, Polyorthoester, Polyphosphate, Polyphosphonate, Polyalkohole, Polyzucker, Polyether, resorbierbare Gläser, Copolymere von polymerisierbaren Substanzen davon.

9. Implantat nach einem der Ansprüche 1 bis 8, **dadurch gekennzeichnet, dass** das Implantat auch mit Ultraschall und/oder in magnetischer Resonanztomographie detektierbar ist.

## Revendications

1. Implant de support local avec une structure basique et flexible à base de polymère et avec des éléments visibles aux rayons X, dans lequel l'implant est flexible dans son ensemble, **caractérisé en ce qu'**au moins une partie des éléments visibles aux rayons X comprend un tube polymère ou une corde qui est rempli(e) au moins partiellement de particules d'une taille d'au plus 2,5 mm formées par une substance visible aux rayons X.

2. Implant selon la revendication 1, **caractérisé en ce que** les éléments visibles aux rayons X sont arrangés en un motif de surface.

3. Implant selon la revendication 1 ou 2, **caractérisé en ce que** la structure basique comprend un polymère non résorbable.

4. Implant selon l'une quelconque des revendications 1 à 3, **caractérisé en ce que** la structure basique a l'une des formes choisies dans le groupe suivant: des filets, des bandes, des feuilles, des feuilles perforées.

5. Implant selon l'une quelconque des revendications 1 à 4, **caractérisé en ce que** les éléments visibles aux rayons X ont au moins l'une des substances visibles aux rayons X choisies dans le groupe suivant: du dioxyde de zirconium pur, du dioxyde de zirconium stabilisé, du nitrure de zirconium, du carbure de zirconium, du tantale, du pentoxyde de tantale, du sulfate de baryum, de l'argent, de l'iodure d'argent, de l'or, du platine, du palladium, de l'iridium, du cuivre, des oxydes ferriques, des aciers pour implant pas très magnétiques, des aciers pour implant non magnétiques, du titane, des iodures alcalins, des composés aromatiques iodés, des composés aliphatiques iodés, des oligomères iodés, des polymères iodés, des alliages de substances de ceux-ci pouvant être alliées.

6. Implant selon l'une quelconque des revendications 1 à 5, **caractérisé en ce que** la substance visible aux rayons X est en outre fixée dans le tube polymère ou la corde, de préférence par rétraction thermique et/ou collage.

7. Implant selon l'une quelconque des revendications 1 à 6, **caractérisé en ce que** l'implant contient au moins l'une des substances choisies dans le groupe suivant: les polyalkylènes, le polypropylène, le polyéthylène, les polyoléfines fluorées, les polytétrafluoroéthylènes, le fluorure de polyvinylidène, les polyamides, les polyuréthanes, les polyisoprènes, les polystyrènes, les polysilicones, les polycarbonates, les polyaryléthercétones, les polyméthacrylates, les polyacrylates, les polyesters aromatiques, les polyimides, les copolymères de substances polymérisables de ceux-ci.

8. Implant selon l'une quelconque des revendications 1 à 7, **caractérisé en ce que** la structure basique a une proportion de polymère résorbable, qui contient de préférence au moins une des substances choisies dans le groupe suivant : les polyhydroxy acides, les polylactides, les polyglycolides, les polyhydroxybutyrates, les polyhydroxyvalérates, les polycaprolactones, les polydioxanones, les oligo- et polyaminoacides synthétiques et naturels, les polyphosphazènes, les polyanhydrides, les polyorthoesters, les polyphosphates, les polyphosphonates, les polyalcools, les polysaccharides, les polyéthers, les verres résorbables, les copolymères de substances polymérisables de ceux-ci.

9. Implant selon l'une quelconque des revendications 1 à 8, **caractérisé en ce que** l'implant est également détectable au moyen de la tomographie par ultrasons et/ou par résonance magnétique.
